(19) Europäisches Patentamt / European Patent Office / Office européen des brevets

(11) **EP 4 076 309 B1**

(12) **EUROPÄISCHE PATENTSCHRIFT**

(45) Veröffentlichungstag und Bekanntmachung des Hinweises auf die Patenterteilung:
**09.04.2025   Patentblatt 2025/15**

(21) Anmeldenummer: **20812010.5**

(22) Anmeldetag: **23.11.2020**

(51) Internationale Patentklassifikation (IPC):
***A61F 9/008*** (2006.01)

(52) Gemeinsame Patentklassifikation (CPC):
**A61F 9/00804; A61F 9/00827**

(86) Internationale Anmeldenummer:
**PCT/EP2020/083041**

(87) Internationale Veröffentlichungsnummer:
**WO 2021/121876 (24.06.2021 Gazette 2021/25)**

(54) **PLANUNGSVORRICHTUNG, REFRAKTIVES LASERSYSTEM UND VERFAHREN ZUR AUTOMATISIERTEN PLANUNG EINER REFRAKTIVEN CHIRURGISCHEN BEHANDLUNG**

PLANNING APPARATUS, REFRACTIVE LASER SYSTEM AND METHOD FOR AUTOMATED PLANNING OF A REFRACTIVE SURGICAL TREATMENT

APPAREIL DE PLANIFICATION, SYSTÈME LASER DE RÉFRACTION ET PROCÉDÉ DE PLANIFICATION AUTOMATISÉE D'UN TRAITEMENT CHIRURGICAL DE RÉFRACTION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2019   DE 102019135509**

(43) Veröffentlichungstag der Anmeldung:
**26.10.2022   Patentblatt 2022/43**

(73) Patentinhaber: **Carl Zeiss Meditec AG
07745 Jena (DE)**

(72) Erfinder:
• **BISCHOFF, Mark
  07745 Jena (DE)**
• **WEYHAUSEN, Andreas
  07745 Jena (DE)**
• **STOBRAWA, Gregor
  07745 Jena (DE)**

(74) Vertreter: **Tautz & Schuhmacher
Nibelungenstraße 84
80639 München (DE)**

(56) Entgegenhaltungen:
EP-A1- 1 719 483            DE-A1- 102008 028 509
DE-A1- 102011 083 928      DE-A1- 102015 218 909

• DAMIEN GATINEL 1 ET AL: "Comparison of the effect of LASIK parameters on the percent tissue altered (1-dimensional metric) versus percent volume altered (3-dimensional metric)", vol. 44, no. 7, 1 July 2018 (2018-07-01), pages 897 - 904, XP009525836, ISSN: 0886-3350, Retrieved from the Internet <URL:https://pubmed.ncbi.nlm.nih.gov/29960656/> [retrieved on 20210223], DOI: 10.1016/J.JCRS.2018.04.041

**Beschreibung**

Die vorliegende Erfindung betrifft eine Planungsvorrichtung, ein refraktives Lasersystem, ein Verfahren zur automatisierten Planung einer refraktiven chirurgischen Behandlung eines Auges mittels eines refraktiven Lasersystems, ein Computerprogrammprodukt und ein computerlesbares Speichermedium. Die Erfindung liegt somit insbesondere auf dem Gebiet der Lasersysteme für ophthalmische, refraktive Chirurgie.

Fehlsichtigkeiten des Auges, insbesondere des menschlichen Auges, können durch verschiedene refraktive chirurgische Behandlungen reduziert oder gar ganz behoben werden. Dazu werden meist refraktive Lasersysteme eingesetzt, mittels welcher die refraktive Wirkung des Auges geändert wird, um auf diese Weise der Fehlsichtigkeit entgegenzuwirken. Insbesondere werden häufig Excimer-Laser verwendet, mit welchen von der Hornhaut bzw. Kornea Gewebe abgetragen wird, um die gewünschte Korrektur der refraktiven Wirkung der Hornhaut zu erzielen. Für ein derartiges Verfahren kann die temporäre Beseitigung eines Flaps erforderlich sein, sodass die darunterliegende Kornea mit dem Excimer-Laser bearbeitet werden kann, wie dies in LASIK-Verfahren erfolgt. Das Lösen des Flaps kann beispielsweise mit einem Femtosekundenlaser erfolgen.

In anderen Verfahren werden refraktive Lasersysteme mit einem Femtosekundenlaser verwendet, um innerhalb der Kornea ein Lentikel herauszulösen, welches sodann in einem chirurgischen Eingriff entfernt werden kann. Durch das Herauslösen des Lentikels wird die refraktive Wirkung der Kornea angepasst, um die Fehlsichtigkeit zu korrigieren. Derartige Verfahren werden als SMILE bezeichnet.

Da die Kornea nur eine endliche Dicke aufweist, ist stets zu beachten, dass der Materialabtrag von der Kornea ein vorbestimmtes Maß nicht übersteigt, da andernfalls Komplikationen an der Hornhaut bzw. am Auge zu befürchten sind. Typischerweise wird daher die Tiefe des Materialabtrags, also die Ausdehnung des Materialabtrags radial zum Mittelpunkt des Auges, ermittelt und mit einem vorbestimmten Höchstwert verglichen. Die Tiefe des Materialabtrags kann insbesondere bei der Planung der refraktiven chirurgischen Behandlung mit dem vorgegebenen Höchstwert verglichen werden. Die Planung erfolgt zumeist automatisiert und computerimplementiert durch eine Planungsvorrichtung, welche beispielsweise Teil des refraktiven Lasersystems sein kann. Sollte die zu erwartende Tiefe des Materialabtrags den Höchstwert übersteigen oder derart groß sein, dass das Risiko von Komplikationen in keinem rechtzufertigenden Verhältnis zur Reduktion der Fehlsichtigkeit steht, kann der Chirurg anhand der Planung der refraktiven chirurgischen Behandlung beispielsweise von der Durchführung der Behandlung abraten und/oder Änderungen anregen oder durchführen.

Die DE 10 2008 028509 A1 beschreibt eine Behandlungsmusterüberwachungsvorrichtung zum Bestimmen einer Anwendbarkeit eines Behandlungsmusters zur Manipulation einer Kornea eines Auges unter Verwendung eines Lasers. Die EP 1 719 483 A1 beschreibt ein Verfahren zur Steuerung eines Lasers für die Ablation einer Hornhautschicht eines Auges. Die DE 10 2011 083 928 A1 beschreibt eine Behandlungsvorrichtung zur operativen Fehlsichtigkeitskorrektur eines Auges und Verfahren zum Erzeugen von Steuerdaten dafür und Verfahren zur operativen Fehlsichtigkeitskorrektur eines Auges. Die DE 10 2015 218909 A1 beschreibt eine Planungseinrichtung zum Erzeugen von Steuerdaten für eine Behandlungsvorrichtung.

Wissenschaftliche Studien widmeten sich bereits der modellhaften Berechnung von Volumenelementen, welche die Kornea charakterisieren und welche von einer refraktiven chirurgischen Behandlung betroffen sind. Beispielsweise beschreibt die Fachpublikation von D. Gantiel et al.: "Comparison of the effect of LASIK parameters on the percent tissue altered (1-dimensionalmetric) versus percent volume altered (3-dimensional metric)" J Cataract Refract Surg 2018; 44:897-904 eine modellhafte Berechnung von Volumenelementen in der Kornea.

Solche modellhaften Berechnungen eignen sich jedoch nicht für eine Verwendung in der Praxis, da zum einen die erforderlichen Berechnungen eine sehr hohe Komplexität aufweisen und ein hohes Maß an mathematischen Kenntnissen und einen erheblichen Zeitaufwand erfordern und dadurch von einem Chirurgen bei der Planung des refraktiven chirurgischen Eingriffs nicht standardmäßig in Vorbereitung auf eine refraktive chirurgische Behandlung durchgeführt werden können. Außerdem basieren derartige Berechnungen lediglich auf einem Standard-Kornea-Modell, welches auf einer vereinfachten geometrischen Näherung einer typischen Kornea-Form basiert. Von diesem Standard-Kornea-Modell weichen tatsächliche Korneae von zu behandelnden Augen oftmals deutlich ab, sodass die dadurch verursachten Abweichungen ein für die chirurgische Behandlung tolerierbares Maß deutlich übersteigen würden.

Es ist daher von zentraler Bedeutung, die zu erwartenden biomechanischen Auswirkungen der refraktiven chirurgischen Behandlung auf das Auge möglichst exakt zu ermitteln. Der Erfindung liegt daher die Aufgabe zugrunde, die automatisierte Planung von refraktiven chirurgischen Behandlungen dahingehend zu verbessern, dass die biomechanischen biomechanischen Auswirkungen bzw. Einwirkungen der refraktiven chirurgischen Behandlung auf das Auge möglichst exakt ermittelt werden können.

Diese Aufgabe wird erfindungsgemäß gelöst durch eine Planungsvorrichtung zur automatisierten Planung einer refraktiven chirurgischen Behandlung, einem Verfahren zur automatisierten Planung einer refraktiven chirurgischen Behandlung eines Auges, einem refraktiven Lasersystem, einem Computerprogrammprodukt und einem computerlesbaren Speichermedium mit den Merkmalen des jeweiligen unabhängigen Anspruchs. Vorteilhafte Ausgestaltungen sind in den Unteransprüchen und in der Beschreibung angegeben.

**In** einem ersten Aspekt betrifft die Erfindung eine Planungsvorrichtung zur automatisierten Planung einer refraktiven chirurgischen Behandlung eines Auges mittels eines refraktiven Lasersystems, wobei die Planungsvorrichtung dazu eingerichtet ist, die folgenden Schritte durchzuführen. In einem Schritt erfolgt ein Planen der refraktiven chirurgischen Behandlung des Auges auf Basis von vorbestimmten Eigenschaften des Auges. In einem weiteren Schritt erfolgt ein Berechnen zumindest einer volumetrischen Kenngröße der Kornea des Auges unter Verwendung der vorbestimmten Eigenschaften des Auges, wobei die volumetrische Kenngröße eine Auswirkung der geplanten refraktiven chirurgischen Behandlung auf das Auge charakterisiert.

In einem weiteren Aspekt betrifft die Erfindung ein Verfahren zur automatisierten Planung einer refraktiven chirurgischen Behandlung eines Auges mittels eines refraktiven Lasersystems. Das Verfahren umfasst ein Planen der refraktiven chirurgischen Behandlung des Auges auf Basis von vorbestimmten Eigenschaften des Auges, sowie ein Berechnen zumindest einer volumetrischen Kenngröße der Kornea des Auges unter Verwendung der vorbestimmten Eigenschaften des Auges, wobei die volumetrische Kenngröße eine Auswirkung der geplanten refraktiven chirurgischen Behandlung auf das Auge charakterisiert.

In einem weiteren Aspekt betrifft die Erfindung ein refraktives Lasersystem zur refraktiven chirurgischen Behandlung eines Auges. Das refraktive Lasersystem weist eine Recheneinheit auf, welche zur Planung einer refraktiven Chirurgischen Behandlung eines Auges mittels des refraktiven Lasersystems und dabei zur Durchführung der folgenden Schritte eingerichtet ist. In einem Schritt erfolgt ein Planen der refraktiven chirurgischen Behandlung des Auges auf Basis von vorbestimmten Eigenschaften des Auges. In einem weiteren Schritt erfolgt ein Berechnen zumindest einer volumetrischen Kenngröße der Kornea des Auges unter Verwendung der vorbestimmten Eigenschaften des Auges, wobei die volumetrische Kenngröße eine Auswirkung der geplanten refraktiven chirurgischen Behandlung auf das Auge charakterisiert.

In einem weiteren Aspekt betrifft die Erfindung ein Computerprogrammprodukt, umfassend Befehle, die bei der Ausführung des Programms durch eine Recheneinheit diese veranlassen, ein erfindungsgemäßes Verfahren auszuführen.

In einem weiteren Aspekt betrifft die Erfindung ein computerlesbares Speichermedium, umfassend Befehle, die bei der Ausführung des Programms durch eine Recheneinheit diese veranlassen, ein erfindungsgemäßes Verfahren auszuführen.

Eine refraktive chirurgische Behandlung kann dabei insbesondere eine Behandlung eines Auges, insbesondere eines menschlichen Auges, sein, beispielsweise um eine Fehlsichtigkeit zu korrigieren. Vorzugsweise wird bei der refraktiven chirurgischen Behandlung die Kornea bzw. Hornhaut hinsichtlich ihrer refraktiven Wirkung geändert, um der diagnostizierten Fehlsichtigkeit zumindest teilweise entgegenzuwirken.

Ein refraktives Lasersystem ist dabei insbesondere eine Vorrichtung, welche zumindest einen Laser umfasst und insbesondere zur Änderung der refraktiven Wirkung der Kornea verwendet werden kann. Bevorzugt verwendet das refraktive Lasersystem einen Laserstrahl, um Gewebe von der Kornea abzutragen und/oder aus der Kornea herauszulösen. Besonders bevorzugt umfasst das refraktive Lasersystem einen Excimer-Laser und/oder einen Femtosekundenlaser. Beispielsweise kann das refraktive Lasersystem einen Excimer-Laser zur Ablation von Gewebe der Kornea und optional einen Femtosekundenlaser zur Sektion des Flaps aufweisen und zur Durchführung einer refraktiven chirurgischen Behandlung gemäß der LASIK Technologie eingerichtet sein. Alternativ oder zusätzlich kann das refraktive Lasersystem einen Femtosekundenlaser aufweisen und dazu eingerichtet sein, refraktive chirurgische Behandlungen gemäß der SMILE Technologie durchzuführen.

Die automatisierte Planung umfasst dabei insbesondere die computerimplementierte Berechnung bzw. Erstellung des Bestrahlungsplans der Hornhaut bzw. Kornea durch das refraktive Lasersystem, um die vom Chirurgen vorgegebene refraktive chirurgische Behandlung durchzuführen. Insbesondere kann die automatisierte Planung eine eigenständige, computerimplementierte Berechnung der Bestrahlung umfassen und die Ausgabe eines entsprechenden Vorschlags für den Behandlungsplan an den Chirurgen bzw. Benutzer des refraktiven Lasersystems umfassen.

Die vorbestimmten Eigenschaften des Auges umfassen insbesondere geometrische Abmessungen des Auges, welche für die Planung und/oder die refraktive chirurgische Behandlung des Auges zur Korrektur der Fehlsichtigkeit erforderlich sind. Insbesondere können die vorbestimmten Eigenschaften des Auges zumindest patientenspezifische Zustandsdaten der Kornea des Auges umfassen. Besonders bevorzugt umfassen die vorbestimmten Eigenschaften des Auges ein Dickenprofil der Kornea und/oder einen Durchmesser der Kornea und/oder einen Krümmungsradius der Kornea und/oder eine Festigkeit der Kornea und/oder ein Epithelprofil der Kornea. Die vorbestimmten Eigenschaften des Auges können beispielsweise durch ein oder mehrere diagnostische Verfahren ermittelt werden. Die diagnostische Ermittlung der vorbestimmten Eigenschaften des Auges kann beispielsweise zeitlich vor der refraktiven chirurgischen Behandlung des Auges durchgeführt werden, sodass die vorbestimmten Eigenschaften des Auges sodann dem refraktiven Lasersystem und/oder der Planungsvorrichtung bereitgestellt werden können. Beispielsweise können die für die refraktive chirurgische Behandlung des Auges erforderlichen Eigenschaften des Auges zumindest teilweise mittels eines OCT-Verfahrens ermittelt werden.

Die Auswirkung der geplanten refraktiven chirurgischen Behandlung auf das Auge, welche durch die zumindest eine volumetrische Kenngröße charakterisiert wird, umfasst insbesondere eine biomechanische Auswirkung der refraktiven chirurgischen Behandlung auf das Auge. Insbesondere sind darunter derartige Auswirkungen zu verstehen, die sich aufgrund eines Volumenabtrags und/oder einer Volumenentnahme aus der Kornea für das Auge ergeben und welche gegebenenfalls das Auge in seiner Funktion und/oder Gesundheit beeinträchtigen können.

Die Erfindung bietet den Vorteil, dass die zu erwartenden biomechanischen Auswirkungen einer geplanten refraktiven chirurgischen Behandlung auf das Auge deutlich besser antizipiert werden können, als dies mit herkömmlichen Verfahren bzw. Planungsvorrichtungen möglich ist. Dadurch lässt sich das Risiko etwaiger durch die geplante refraktive chirurgische Behandlung zuverlässiger einschätzen, wodurch eine bessere Abwägung der möglichen Risiken gegenüber dem zu erwartenden Vorteil hinsichtlich der Korrektur der Fehlsichtigkeit ermöglicht wird. Somit bietet die Erfindung den Vorteil, dass einem Chirurgen die Möglichkeit gegeben wird, eine genauere Prognose und damit einhergehend eine bessere Beratung der Patienten anzubieten.

Insbesondere bietet die Erfindung den Vorteil, dass durch das Berechnen der volumetrischen Kenngröße die Auswirkung der geplanten refraktiven chirurgischen Behandlung auf das Auge bzw. auf die Kornea deutlich besser antizipiert bzw. eingeschätzt werden kann, als dies bei herkömmlichen Verfahren unter Verwendung einer maximalen Ablationstiefe möglich ist. Insbesondere ermöglicht die Erfindung, eine anteilige und/oder prozentuale Volumenänderung der Kornea zu antizipieren, anhand welcher sich die Risiken für etwaige Komplikationen deutlich besser erkennen lassen, als bei herkömmlichen Verfahren.

Ferner bietet die Erfindung den Vorteil, dass sich das Berechnen der zumindest einen volumetrischen Kenngröße und damit einhergehend die optionale Beurteilung des Risikos von etwaigen Komplikationen zusammen mit der Planung der refraktiven chirurgischen Behandlung kombinieren lassen und/oder in die Planung mit einbinden lassen. Auf diese Weise kann insbesondere das Berechnen der volumetrischen Kenngröße auf voll-automatisierte Weise erfolgen, ohne dass dazu eine aufwändige und komplexe Berechnung durch den Chirurgen erforderlich ist. Somit wird durch die Erfindung der Chirurg zum einen entlastet und erhält dennoch durch das erfindungsgemäße Verfahren eine bedeutende Entscheidungshilfe zur Beurteilung der etwaigen Risiken, die sich aus der geplanten refraktiven chirurgischen Behandlung ergeben.

Bevorzugt basiert die zumindest eine volumetrische Kenngröße auf zumindest einem der folgenden Volumenelemente: ein von der Kornea ablatiertes und/oder exzidiertes Volumen, ein Flap-Volumen, ein aus der Kornea gelöstes Volumen, ein Lentikelvolumen, ein von der Kornea ablatiertes und/oder exzidiertes bzw. aus der Kornea gelöstes Teilvolumen des Epithels und/oder des Stromas und/oder des Endothels und/oder ein aus der Kornea gelöstes oder von der Kornea ablatiertes und/oder exzidiertes nach dessen Festigkeit gewichtetes Volumen und/oder ein in die Kornea implantiertes Volumen. Bei einem von der Kornea ablatierten und/oder exzidierten Volumen kann es sich insbesondere um solch ein Volumen der Kornea handeln, welches durch einen Excimer-Laser bei der refraktiven chirurgischen Behandlung des Auges abgetragen wird. Bei einem Lentikelvolumen kann es sich insbesondere um einen Teil der Kornea handeln, welches im Rahmen eines SMILE Verfahrens mittels eines Femtosekundenlasers aus der Kornea herausgelöst und mittels eines chirurgischen Eingriffs entnommen wird bzw. werden soll.

Die Berücksichtigung einer oder mehrerer derartiger Volumenelemente als volumetrische Kenngröße bietet den Vorteil, dass die biomechanische Auswirkung des refraktiven chirurgischen Eingriffs, welcher eben jene volumetrische Kenngröße betrifft, besonders genau und zuverlässig antizipiert und/oder beurteilt werden kann.

Das Verfahren umfasst ferner ein Berechnen einer zu erwartenden Volumenänderung der Kornea aufgrund der geplanten refraktiven chirurgischen Behandlung des Auges. Insbesondere kann die Volumenänderung als eine anteilsmäßige Volumenänderung und/oder eine prozentuale Volumenänderung des Volumens der Kornea berechnet werden. Daraus wird eine PVA Kennzahl (Percent Volume Altered) bestimmt, welche in wissenschaftlichen Studien als besonders geeignet für eine Beurteilung der biomechanischen Auswirkungen der chirurgischen Behandlung auf das Auge angesehen wird. Dabei kann unter anderem auch von Interesse sein, welcher Volumenanteil der Kornea nach der refraktiven chirurgischen Behandlung noch im Auge verbleibt.

Das Verfahren umfasst ferner ein Ausgeben der PVA Kennzahl und vorzugsweise der zu erwartenden Volumenänderung der Kornea und/oder einer darauf basierenden Information an einen Benutzer des refraktiven Lasersystems und/oder der Planungsvorrichtung. Dies bietet den Vorteil, dass einem Chirurgen und/oder einem Benutzer des refraktiven Lasersystems die ermittelten Informationen bereitgestellt werden können, sodass dieser die gewonnenen Informationen für eine Beurteilung des Risikos und/oder für die Beratung des Patienten nutzbringend verwenden kann.

Vorzugsweise umfasst das Verfahren ferner ein automatisiertes Beurteilen der refraktiven chirurgischen Behandlung des Auges anhand der zumindest einen volumetrischen Kenngröße. Dies bietet den Vorteil, dass bereits computerimplementiert und automatisiert eine Auswertung der erfassten volumetrischen Kenngröße erfolgen kann und eine Beurteilung der refraktiven chirurgischen Behandlung, bevorzugt des Risikos, ohne weiteres Zutun des Chirurgen und/oder des Benutzers erfolgen kann.

Vorzugsweise umfasst das Verfahren ferner ein Bereitstellen eines Ergebnisses der automatisierten Beurteilung der refraktiven chirurgischen Behandlung des Auges an einen Benutzer des refraktiven Lasersystems und/oder der Planungsvorrichtung. Dies bietet den Vorteil, dass dem Chirurgen und/oder Benutzer des refraktiven Lasersystems eine wertvolle Information bereitgestellt werden kann, anhand welcher dieser auf etwaige Risiken hingewiesen wird und/oder bei seiner eigenen Beurteilung der Risiken unterstützt wird. Insbesondere ermöglicht es dem Chirurgen oder Benutzer des refraktiven Lasersystems, ohne persönlichen Mehraufwand eine fundiertere Beurteilung der refraktiven chirurgischen Behandlung und der damit verbundenen Risiken zu treffen.

Vorzugsweise umfasst das automatisierte Beurteilen der refraktiven chirurgischen Behandlung ein Beurteilen eines Risikos für das Auge aufgrund einer Änderung des Volumens der Kornea durch die refraktive chirurgische Behandlung des Auges. Dabei umfasst das Verfahren optional ein Ausgeben einer Information auf Basis des beurteilten Risikos an den Benutzer des refraktiven Lasersystems und/oder der Planungsvorrichtung, und/oder ein automatisiertes Anpassen der geplanten refraktiven Chirurgischen Behandlung zur Reduktion des Risikos und/oder Vorschlagen einer Anpassung der geplanten refraktiven chirurgischen Behandlung zur Reduktion des Risikos, sofern das Risiko ein erstes vorbestimmtes Maß übersteigt. Optional umfasst das Verfahren ein Unterbinden einer Durchführung der refraktiven chirurgischen Behandlung, sofern das beurteilte Risiko ein zweites vorbestimmtes Maß übersteigt. Dies bietet den Vorteil, dass der Chirurg und/oder der Benutzer des refraktiven Lasersystems bei der Beurteilung und/oder der Entscheidung hinsichtlich der Durchführung des refraktiven chirurgischen Verfahrens unterstützt werden und auf diese Weise die Gefahr verringert wird, dass etwaige Risiken unberücksichtigt bleiben.

Vorzugsweise umfasst das Verfahren ferner ein Ermitteln eines Zusammenhanges der zumindest einen volumetrischen Kenngröße mit einem Ergebnis der refraktiven chirurgischen Behandlung des Auges. Dies bietet den Vorteil, dass das Verfahren zur automatisierten Planung und insbesondere zum Berechnen der volumetrischen Kenngröße überprüft werden kann und/oder sich selbst überprüfen kann und auf diese Weise seine Funktionalität überprüfen und/oder verbessern kann. Insbesondere ermöglicht dies, die automatisierte Planung und/oder das Berechnen der volumetrischen Kenngröße als lernfähiges Computerprogramm auszugestalten. Insbesondere kann gemäß bevorzugter Ausführungsformen postoperativ ein Zusammenhang des erzielten Ergebnisses durch die erhöhte refraktive chirurgische Behandlung und die Berechnung der volumetrischen Kenngröße vor der Behandlung analysiert werden. Dies kann beispielsweise mittels statistischer Auswertung einer Vielzahl von Datensätzen, beispielsweise von mehr als 100 oder gar mehr als 1000 Datensätzen, erfolgen, sodass daraus Anpassungen für die Berechnung der volumetrischen Kenngröße und/oder der automatisierten Planung, wie etwa Parameterbereiche und/oder Klassifizierungsregeln, abgeleitet werden können. Dieser Zusammenhang kann sodann beispielsweise auch bei zukünftigen Behandlungsplanungen berücksichtigt werden. Beispielsweise kann die ermittelte volumetrische Kenngröße in einem Behandlungsprotokoll, beispielsweise im refraktiven Lasersystem und/oder extern auf einem Server zentralisiert archiviert werden und vorzugsweise im Zusammenhang mit anderen Parametern für automatisierte Lernprozesse verwendet werden.

Die Merkmale, welche für das Verfahren beschrieben sind, sind in gleicher Weise für eine Planungsvorrichtung, welche zur Durchführung eines solchen Verfahrens ausgelegt ist, als offenbart anzusehen. Zugunsten der Knappheit werden diese nicht im Einzelnen wiederholt.

Vorzugsweise weist das refraktive Lasersystem zumindest einen Femtosekundenlaser auf, vorzugsweise zum refraktiven chirurgischen Herauslösen eines Lentikels aus der Kornea im Rahmen eines SMILE Verfahrens und/oder zur Präparation eines Flaps des Auges im Rahmen eines LASIK Verfahrens. Alternativ oder zusätzlich weist das refraktive Lasersystem vorzugsweise zumindest einen Excimer-Laser auf, vorzugsweise zur Ablation der Kornea.

Ein Computerprogrammprodukt kann beispielsweise in Form eines Programmcodes vorliegen, welche auf einem physikalischen Datenträger gespeichert ist und/oder über ein Netzwerk heruntergeladen werden kann.

Die oben genannten und im Folgenden erläuterten Merkmale und Ausführungsformen sind dabei nicht nur als in den jeweils explizit genannten Kombinationen offenbart anzusehen, sondern sind auch in anderen technisch sinnhaften Kombinationen und Ausführungsformen vom Offenbarungsgehalt umfasst.

Die Erfindung wird durch die Ansprüche definiert.

Weitere Einzelheiten und Vorteile der Erfindung sollen nun anhand von den folgenden Beispielen und bevorzugten Ausführungsformen mit Bezug auf die Figuren näher erläutert werden.

Es zeigen:

Fig. 1          ein refraktives Lasersystem für die refraktive Chirurgie eines Auges gemäß einer bevorzugten Ausführungsform der Erfindung;

Fig. 2A und 2B          Illustrationen zur Erläuterung einer beispielhaften Berechnung eines aus der Kornea zu entfernenden bzw. entfernten Volumens zur Korrektur einer rein sphärischen Fehlsichtigkeit;

Fig. 3A und 3B          Illustrationen zur Erläuterung einer beispielhaften Berechnung einer volumetrischen Kenngröße, welche Flap und/oder Cap darstellt;

Fig. 4          Illustrationen zur Erläuterung einer beispielhaften Berechnung einer volumetrische Kenngröße, die dem Volumen des entfernten Korneagewebes für die Korrektur einer sphärozylindrischen Fehlsichtigkeit entspricht.

In den folgenden Figuren werden gleiche oder ähnliche Elemente in den verschiedenen Ausführungsformen der Einfachheit halber mit gleichen Bezugszeichen bezeichnet.

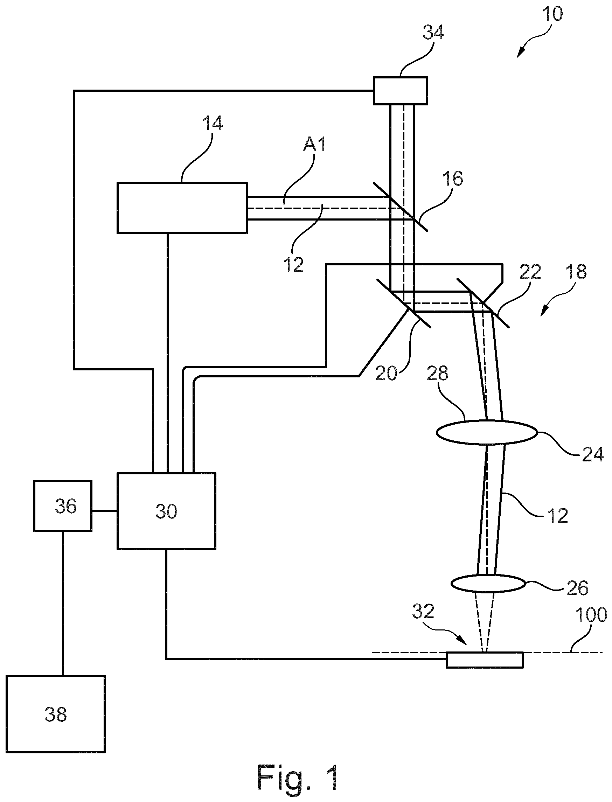

Figur 1 zeigt ein refraktives Lasersystem 10 für die refraktive Chirurgie eines Auges gemäß einer bevorzugten Ausführungsform der Erfindung. Das refraktive Lasersystem 10 ist als ein Behandlungsgerät ausgebildet und dient beispielsweise dazu, an einem Auge eines Patienten (nicht gezeigt) eine Fehlsichtigkeitskorrektur mittels eines Verfahrens zur refraktiven Chirurgie unter Verwendung eines Laserstrahls 12 bzw. Bearbeitungslaserstrahls 12 auszuführen. Dazu weist das refraktive Lasersystem 10 einen Laser bzw. eine Laserquelle 14 auf, welche den Laserstrahl 12 emittiert. Der Laserstrahl 12 ist dazu ausgebildet, auf die Hornhaut eines Auges einzuwirken, um die refraktive Wirkung der Hornhaut zu verändern.

Der vom Laser 14 entlang einer optischen Achse A1 abgegebene Laserstrahl 12 bzw. Bearbeitungsstrahl 12 fällt dabei auf einen Strahlteiler 16, der den Laserstrahl 12 auf eine Ablenkeinrichtung 18 ausgebildete Ablenkeinheit 18 leitet. Die Ablenkeinheit 18 weist zwei Scanspiegel 20 und 22 auf, die um zueinander orthogonale Achsen drehbar sind, so dass die Ablenkeinheit 18 den Laserstrahl 12 zweidimensional ablenkt. Eine verstellbare Projektionsoptik 24 fokussiert den Laserstrahl 12 zur Bearbeitung auf das Bearbeitungsobjekt bzw. auf bzw. in ein zu behandelndes Auge. Die Projektionsoptik 24 weist dabei zwei Linsen 26 und 28 auf. Das zu behandelnde Auge kann zur Behandlung in der Arbeitsebene 100 angeordnet werden, sodass der Laserstrahl 12 darauf fokussiert werden kann.

Ferner weist das refraktives Lasersystem 10 eine Steuereinheit 30 auf. Die Steuereinheit 30 bestimmt vorzugsweise die Lage des Fokus 32 sowohl senkrecht zur optischen Achse A1 (durch die Scanspiegel 20 und 22) sowie in Richtung der optischen Achse A1. Die Steuereinheit 30 liest weiter einen Detektor 34 aus, der beispielsweise als Mitbeobachtungseinheit agiert und zur Überwachung des Bearbeitungsvorgangs dient. Zusätzlich kann das refraktive Lasersystem 10 weitere Sensoren und/oder Detektoren aufweisen, insbesondere einen internen Energiesensor bzw. Energiedetektor, die jedoch in der Figur nicht gezeigt sind. Beispielsweise kann der Energiesensor hinter dem Strahlteiler 16 angeordnet sein, um die durch den Strahlteiler 16 transmittierte Energie des Laserstrahls 12 zu ermitteln.

Zudem weist das refraktive Lasersystem 10 eine Planungsvorrichtung 36 auf. Die Planungsvorrichtung 36 ist vorzugsweise als eine Recheneinheit ausgebildet. Gemäß einer bevorzugten Ausführungsform können die Steuereinheit 30 und die Planungsvorrichtung 36 in eine einzige Recheneinheit integriert sein oder durch eine einzige Recheneinheit ausgebildet sein. Gemäß der gezeigten Ausführungsform ist jedoch die Planungsvorrichtung 36 separat von der Steuereinheit 30 ausgebildet. Die Planungsvorrichtung 36 kann mit der Steuereinheit 30 verbunden sein und ist vorzugsweise auch mit einem Ein- und Ausgabegerät 38 verbunden, etwa mit einem Display und einer Tastatur und/oder einem Mauseingabegerät, über welche Informationen an den Benutzer ausgegebene werden können und Befehle vom Benutzer entgegengenommen werden können.

Die Planungsvorrichtung 36 dient zur Planung der refraktiven chirurgischen Behandlung von Augen mittels des refraktiven Lasersystems 10. Dabei ist die Planungsvorrichtung 36 dazu eingerichtet, die refraktiven chirurgischen Behandlung des Auges auf Basis von vorbestimmten Eigenschaften des Auges zu planen. Die vorbestimmten Eigenschaften des zu behandelnden Auges können beispielsweise vom Benutzer oder von einer zentralen Stelle, etwa einem Server, bereitgestellt werden, sodass die Planungsvorrichtung diese nutzen kann. Die Planungsvorrichtung ist ferner dazu eingerichtet, zumindest eine volumetrischen Kenngröße der Kornea des Auges unter Verwendung der vorbestimmten Eigenschaften des Auges, wobei die volumetrische Kenngröße eine Auswirkung der geplanten refraktiven chirurgischen Behandlung auf das Auge charakterisiert, zu berechnen.

Über das Ein- und Ausgabegerät 38 kann die Planungsvorrichtung 36 sodann dem Benutzer Informationen über die Planung der Behandlung ausgeben und insbesondere auf etwaige erkannte Risiken, die anhand der volumetrischen Kenngröße ermittelt wurden, hinweisen. Der Benutzer kann sodann beispielsweise über einen entsprechenden Befehl den Behandlungsplan anpassen oder, sofern zu hohe Risiken vorliegen, von der Behandlung absehen. Sollten keine Risiken erkannt worden sein, welche eine Änderung des Behandlungsplans erfordern oder die Behandlung als nicht durchführbar erscheinen lassen, kann der Benutzer beispielsweise durch die Eingabe eines entsprechenden Befehls die Durchführung der Behandlung gemäß Behandlungsplan bewirken. Dazu kann beispielsweise die Planungsvorrichtung 36 entsprechende Befehle und/oder Informationen an die Steuereinheit 30 übermitteln, sodass diese die Behandlung gemäß dem Behandlungsplan durchführen kann.

Anhand der folgenden Figuren werden beispielhaft Algorithmen für die Berechnung verschiedener volumetrischer Kenngrößen beschrieben, derer sich beispielsweise eine Planungsvorrichtung 36 zur Berechnung der zumindest einen volumetrischen Kenngröße bedienen kann. Die Erfindung ist jedoch nicht auf die folgenden Algorithmen beschränkt.

Beispiel 1

In Beispiel 1 wird die Berechnung eines aus der Kornea zu entfernenden bzw. entfernten Volumens zur Korrektur einer rein sphärischen Fehlsichtigkeit erläutert.

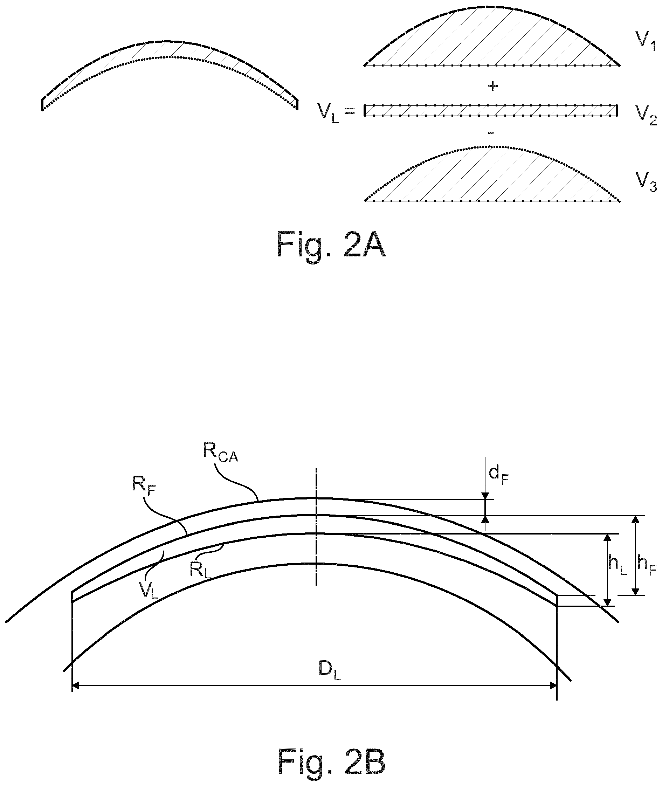

Das Volumen des zu entfernenden Gewebes $V_L$ mit dem Durchmesser $D$ kann unter Verwendung von drei Blöcken berechnet werden, wie in Figur 2 gezeigt. Im vorliegenden Beispiel werden zwei sphärische Elemente benutzt, welche durch eine innere und eine äußere Oberfläche definiert sind, sowie ein Zylinderelement, welches dem Volumenelement einen Sockel hinzufügt.

Das resultierende Volumen des Volumenelements kann sodann durch die folgende Formel berechnet werden:

$$V_L = V_1 + V_2 - V_3. \qquad (A.1)$$

Zur Berechnung der Teilvolumina $V_1$ und $V_3$ kann die Formel für eine sphärische Kuppel herangezogen werden:

$$V = \frac{\pi}{6} h \left( 3 \left( \frac{D}{2} \right)^2 + h^2 \right), \quad (A.2)$$

wobei D den Durchmesser der Kuppel und h die Höhe darstellen. Das Volumen des zylindrischen Teilelements $V_2$ kann wie folgt berechnet werden:

$$V = \frac{\pi}{4} D_L^2 d, \qquad (A.3)$$

wobei D wiederum den Durchmesser darstellt und d die Höhe.

Bei einer rein sphärischen Korrektur, wie im vorliegenden Fall, stellen sich die Parameter, die das zu entfernenden Gewebe definieren, gemäß Figur 2B dar.

Durch Einsetzen dieser Parameter in die obigen Formeln erhält man:

$$V_1 = \frac{\pi}{6} h_F \left( 3 \left( \frac{D_L}{2} \right)^2 + h_F^2 \right), \qquad (A.4)$$

$$V_3 = \frac{\pi}{6} h_L \left( 3 \left( \frac{D_L}{2} \right)^2 + h_L^2 \right). \qquad (A.5)$$

Der Durchmesser $D_L$ entspricht dabei dem Durchmesser eines Lentikels, welcher im Rahmen einer SMILE Behandlung entfernt wird, oder dem Durchmesser des Volumens, welches im Rahmen einer PRK oder LASIK Behandlung mittels eines Excimer-Laser ablatiert wird.

Die Höhe der sphärischen Kuppeln kann durch die Krümmungsradien der inneren und äußeren Oberflächen ausgedrückt werden:

$$h_F = R_F - \sqrt{R_F^2 - \frac{D_L^2}{4}}, \qquad (A.6)$$

$$R_F = R_{CA} - d_F \qquad , \qquad (A.7)$$

$$h_L = R_L - \sqrt{R_L^2 - \frac{D_L^2}{4}}, \qquad (A.8)$$

$$R_L = R_{CA}^* - d_F \qquad . \qquad (A.9)$$

Für eine SMILE Behandlung sind diese Oberflächen durch den Cap-Schnitt und den Lentikel Schnitt vorgegeben. Für LASIK Behandlungen werden diese Oberflächen durch den Flap-Schnitt und die stromalen Bodenschnittoberfläche nach der Ablation durch den Excimer-Laser vorgegeben.

Für eine PRK Behandlung behalten diese Gleichungen ihre Gültigkeit mit einer Epitheldicke von $d_{Epithelium} = d_F$.

Im allgemeinen ist festzustellen, dass für eine korneale Laserfehlsichtigkeitskorrektur die postoperative Krümmung der Kornea anhand der präoperativen Krümmung der Kornea, der Brechtkraft vor der Operation und dem Brechungsindex der Kornea berechnet werden kann:

$$R_{CA}^* = \left( \frac{1}{R_{CA}} + \frac{B_{CL}}{(n_C - 1)} \right)^{-1}. \qquad (A.10)$$

Für das zylindrische Teilvolumen $V_2$ kann die Höhe $d_{LX}$ verwendet werden, welche einer Sockelhöhe des zu entfernenden Gewebes entspricht, das heißt einer Mindestdicke des Lentikels bei einer SMILE Behandlung oder einem Sockel des zu ablatierenden Volumens bei der Behandlung mit einem Excimer-Laser:

$$V_2 = \frac{\pi}{4} D_L^2 d_{LX}. \qquad (A.11)$$

Durch Kombination der Gleichungen A.4, A.5, und A.11 zur Substitution in Gleichung A.1, erhält man eine kompakte Formel für das zu entfernende Volumenelement für eine rein sphärische Korrektur:

$$V_L = \frac{\pi}{8} D_L^2 \cdot (h_F - h_L + 2d_{LX}) + \frac{\pi}{6} (h_F^3 - h_L^3). \qquad (A.12)$$

Beispiel 2

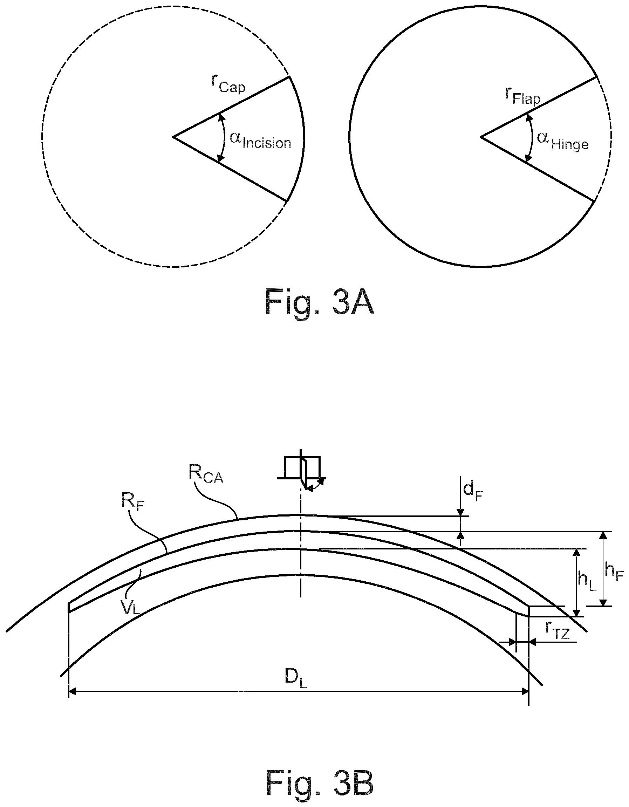

In Beispiel zwei wird mit Bezug auf die Figuren 3A und 3B die Berechnung einer volumetrischen Kenngröße beschrieben, welche ein Flap und/oder Cap darstellt.

Das Volumen $V_A$ einer äußeren zirkularen Lamelle der Kornea mit dem Krümmungsradius $R_{CA}$, dem Durchmesser $r_A$ und der Dicke $d_A$ wird berechnet unter Verwendung einer Näherung, welche in der in der Einleitung zitierten Publikationen von Gatinel et al. beschrieben ist:

$$V_A = \pi \left[ r_A^2 + \left( R_{CA} - \sqrt{R_{CA}^2 - r_A^2} \right)^2 \right] \cdot d_A. \qquad (B.1)$$

Die Geometrie für SMILE Verfahren ist dabei in Figur 3A auf der linken Seite dargestellt. Die Geometrie für LASIK Verfahren ist dabei in Figur 3A auf der rechten Seite dargestellt.

Mit dem Winkel $\alpha$ kann das gelöste Flapvolumen (LASIK Verfahren), welches nach der refraktiven chirurgischen Behandlung nicht mehr im wesentlichen Maße zur Stabilität der Kornea beiträgt, wie folgt berechnet werden:

$$V_{Flap} = V_A \cdot \left[ 1 - \frac{\alpha_{Hinge} - \sin(\alpha_{Hinge})}{2\pi} \right]. \qquad (B.2)$$

Das gelöste Kuppelvolumen in einer SMILE Behandlung kann auf ähnliche Weise wie folgt berechnet werden, mit dem Einschnittwinkel $\alpha$:

$$V_{Cap} = V_A \cdot \frac{\alpha_{Incision} - \sin(\alpha_{Incision})}{2\pi}. \qquad (B.3)$$

Durch Substitution der Formel B.1 in Formel B.2 und mit dem eingesetzten Flapradius $r_{Flap}$, dem Winkel $\alpha_{Hinge}$, und der Flapdicke $d_{Flap}$ erhält man die folgende Formel für das gelöste Flap-Volumen in einem LASIK-Verfahren:

$$V_{Flap} = \pi \left( r_{Flap}^2 + \left[ R_{CV} - \sqrt{R_{CV}^2 - r_{Flap}^2} \right]^2 \right) \cdot d_{Flap} \cdot \left[ 1 - \frac{\alpha_{Hinge} - \sin(\alpha_{Hinge})}{2\pi} \right]. $$

$$(B.4)$$

Das gelöste Capvolumen in einem SMILE Verfahren kann auf ähnliche Weise berechnet werden. Mit dem Capradius $R_{Cap}$ , den Einschnittwinkel $\alpha_{Incision}$, und der Capdicke $d_{Cap}$ erhält man:

$$V_{Cap} = \pi \left( r_{Cap}^2 + \left[ R_{CV} - \sqrt{R_{CV}^2 - r_{Cap}^2} \right]^2 \right) \cdot d_{Cap} \cdot \left[ \frac{\alpha_{Incision} - \sin(\alpha_{Incision})}{2\pi} \right].$$

$$(B.5)$$

Vorteilhafterweise wird der Dickenparameter in den obigen Gleichungen modifiziert, um auszuschließen, dass die Epithelschicht bei der Bestimmung des gelösten Gewebes mit berücksichtigt wird. Dies kann durch eine Subtraktion der durchschnittlichen Epitheldicke von der Flapdicke und der Capdicke erreicht werden:

$$d_{Flap}^* = d_{Flap} - d_{Epithelium} \quad (B.6)$$

$$d_{Cap}^* = d_{Cap} - d_{Epithelium} \quad (B.7)$$

Eine Substitution von $d_{Flap}$ mit $d_{Flap}^*$ , bzw. $d_{Cap}$ mit $d_{Cap}^*$ , führt zum Volumen des Flapschnitts (in LASIK Verfahren) geänderten stromalen Gewebes $V_{Flap}^*$ oder des Flapschnitts (in SMILE Verfahren) $V_{Cap}^*$ .

Beispiel 3

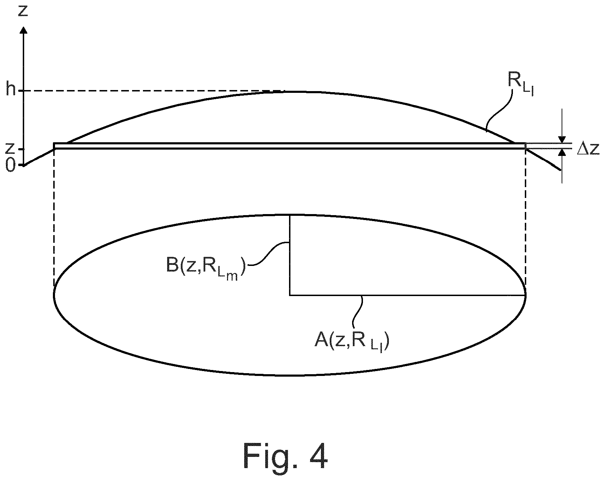

In Beispiel 3 wird mit Bezug auf Figur 4 beispielhaft eine volumetrische Kenngröße berechnet die dem Volumen des entfernten Korneagewebes für die Korrektur einer sphärozylindrischen Fehlsichtigkeit entspricht.

Während ein Lentikel für eine rein sphärische myopische Korrektur durch eine sphärische innere Oberfläche und eine sphärische äußere Oberfläche genähert werden kann, weist ein Lentikel für eine sphärozylindrische myopische Korrektur zumindest eine torische Oberfläche auf (siehe Figur 4).

In diesem Beispiel wird eine sphärische innere Oberfläche und eine torische äußere Oberfläche angenommen, was eine gute Näherung der Situation bei refraktiven chirurgischen Behandlungen von astigmatischer Myopie mittels SMILE-Verfahren darstellt.

Diesem Ansatz folgend kann das zu entfernende Gewebevolumen $V_L$ analog zur Gleichung A.1 berechnet werden, mit dem Unterschied, dass eine sphärische Kuppel und eine torische Kuppel und zusätzlich das zylindrische Volumen aufgrund der Mindestdicke des Lentikels verwendet werden:

$$V_L = V_1 + V_2 - V_3.\qquad (C.1)$$

Während die Berechnung der Teilvolumina $V_1$ and $V_2$ unverändert bleibt gegenüber der rein sphärischen Korrektur (vgl. A.4 und A.11), wird das Teilvolumen $V_3$
der torischen Kuppel durch Lösen des folgenden Integrals berechnet:

$$V_3 = \pi \int_0^h \sqrt{R_{L_m}{}^2 - \left(R_{L_m} - z\right)^2} \sqrt{R_{L_l}{}^2 - \left(R_{L_l} - z\right)^2} dz. \qquad (C.2)$$

Diese Gleichung ist wie folgt zu verstehen: eine Skizze der Herleitung des obigen Integrals ist in Figur 4 gezeigt.

Das Volumen ist in Zylinderscheiben zerlegt, mit einer Ellipse als Grundfläche und einer Höhe $\Delta z$. Das Volumen einer solchen Zylinderscheibe ergibt sich wie folgt:

$$V = \pi \, A \, B \, \Delta z \qquad (C.3)$$

Wobei A die größere Halbachse und B die kleinere Halbachse darstellt. Die Größe dieser Achsen bzw. Halbachsen hängt von der Position in z-Richtung des Zylinders ab, sowie dem Krümmungsradius der torischen Kuppel entlang des Hauptmeridians:

$$A = \sqrt{R_{L_l}{}^2 - \left(R_{L_l} - z\right)^2} \qquad (C.4)$$

$$B = \sqrt{R_{L_m}{}^2 - \left(R_{L_m} - z\right)^2} \qquad (C.5)$$

Das Volumen der torischen Kuppel kann durch die Summe aller Zylinderscheiben genährt werden:

$$V_3 \approx \pi \sum \sqrt{R_{L_l}{}^2 - \left(R_{L_l} - z\right)^2} \sqrt{R_{L_m}{}^2 - \left(R_{L_m} - z\right)^2} \, \Delta z \qquad (C.6)$$

Im Grenzwert von infinitesimal kleinen Höhen der Zylinderscheiben, $\Delta z \to dz$, erhält man das oben genannte Integral (C.3.). Dieses Integral kann durch numerische Integration berechnet werden.

**Bezugszeichenliste**

| | |
|---|---|
| 10 | refraktives Lasersystem |
| 12 | Laserstrahl |
| 14 | Laserquelle |
| 16 | Strahlteiler |
| 18 | Ablenkeinheit |
| 20, 22 | Scanspiegel |
| 24 | Projektionsoptik |
| 26, 28 | Linsen |
| 30 | Steuereinheit |
| 32 | Fokus |
| 34 | Detektor |
| 36 | Planungsvorrichtung |
| 38 | Ein- und Ausgabegerät |
| 100 | Arbeitsebene |

**Patentansprüche**

1. Planungsvorrichtung (36) zur automatisierten Planung einer refraktiven chirurgischen Behandlung eines Auges mittels eines refraktiven Lasersystems (10), wobei die Planungsvorrichtung (36) dazu eingerichtet ist, die folgenden Schritte durchzuführen:

   - Planen der refraktiven chirurgischen Behandlung des Auges auf Basis von vorbestimmten Eigenschaften des Auges;
   - Berechnen zumindest einer volumetrischen Kenngröße der Kornea des Auges unter Verwendung der vorbestimmten Eigenschaften des Auges, wobei die volumetrische Kenngröße eine Auswirkung der geplanten refraktiven chirurgischen Behandlung auf das Auge charakterisiert;
   - Berechnen einer zu erwartenden Volumenänderung der Kornea aufgrund der geplanten refraktiven chirurgischen Behandlung des Auges;

   **dadurch gekennzeichnet, dass** die Planungsvorrichtung (36) ferner dazu eingerichtet ist, die folgenden Schritte durchzuführen:

   - Bestimmen einer Percent-Volume-Altered Kennzahl, PVA Kennzahl, aus der berechneten, zu erwartenden Volumenänderung der Kornea; und
   - Ausgeben der PVA Kennzahl an einen Benutzer des refraktiven Lasersystems und/oder der Planungsvorrichtung.

2. Planungsvorrichtung gemäß Anspruch 1, wobei das Berechnen der zu erwartenden Volumenänderung der Kornea ein Berechnen einer anteilsmäßigen Volumenänderung und/oder einer prozentualen Volumenänderung des Volumens der Kornea umfasst.

3. Planungsvorrichtung (36) gemäß Anspruch 1 oder 2, wobei die zumindest eine volumetrische Kenngröße auf zumindest einer der folgenden Volumenelemente basiert:

  - ein Flap-Volumen;
  - ein von der Kornea ablatiertes und/oder exzidiertes Volumen;
  - ein aus der Kornea gelöstes oder von der Kornea ablatiertes und/oder exzidiertes nach dessen Festigkeit gewichtetes Volumen;
  - ein von der Kornea ablatiertes und/oder exzidiertes bzw. aus der Kornea gelöstes Teilvolumen des Epithels und/oder des Stromas und/oder des Endothels;
  - ein aus der Kornea gelöstes Volumen;
  - ein Lentikelvolumen;
  - ein in die Kornea implantiertes Volumen.

4. Planungsvorrichtung gemäß einem der vorhergehenden Ansprüche, wobei das Planen der refraktiven chirurgischen Behandlung des Auges auf Basis von vorbestimmten Eigenschaften des Auges das Planen eines refraktiven chirurgischen Herauslösens eines Lentikels aus der Kornea im Rahmen eines SMILE Verfahrens umfasst.

5. Planungsvorrichtung (36) gemäß einem der vorhergehenden Ansprüche, wobei die vorbestimmten Eigenschaften des Auges zumindest patientenspezifische Zustandsdaten der Kornea des Auges umfassen und wobei die patientenspezifischen Zustandsdaten optional einen Durchmesser der Kornea und/oder einen Krümmungsradius der Kornea und/oder eine Festigkeit der Kornea und/oder ein Epithelprofil der Kornea umfassen.

6. Planungsvorrichtung (36) gemäß Anspruch 5, wobei die patientenspezifischen Zustandsdaten ein Dickenprofil der Kornea umfassen.

7. Planungsvorrichtung (36) gemäß einem der vorhergehenden Ansprüche, wobei die Planungsvorrichtung (36) dazu eingerichtet ist, ferner den folgenden Schritt durchzuführen:

  - automatisiertes Beurteilen der refraktiven chirurgischen Behandlung des Auges anhand der zumindest einen volumetrischen Kenngröße; und
  - Bereitstellen eines Ergebnisses der automatisierten Beurteilung der refraktiven chirurgischen Behandlung des Auges an einen Benutzer des refraktiven Lasersystems und/oder der Planungsvorrichtung.

8. Planungsvorrichtung (36) gemäß Anspruch 7, wobei das automatisierte Beurteilen der refraktiven chirurgischen Behandlung vorzugsweise ein Beurteilen eines Risikos für das Auge aufgrund einer Änderung des Volumens der Kornea durch die refraktive chirurgische Behandlung des Auges umfasst, und wobei die Planungsvorrichtung (36) optional dazu eingerichtet ist, ferner einen oder mehrere der folgenden Schritte durchzuführen:

  - Ausgeben einer Information auf Basis des beurteilten Risikos an den Benutzer des refraktiven Lasersystems (10) und/oder der Planungsvorrichtung (36);
  - automatisiertes Anpassen der geplanten refraktiven Chirurgischen Behandlung zur Reduktion des Risikos und/oder Vorschlagen einer Anpassung der geplanten refraktiven chirurgischen Behandlung zur Reduktion des Risikos, sofern das Risiko ein erstes vorbestimmtes Maß übersteigt;
  - Unterbinden einer Durchführung der refraktiven chirurgischen Behandlung, sofern das beurteilte Risiko ein zweites vorbestimmtes Maß übersteigt.

9. Refraktives Lasersystem (10) zur refraktiven chirurgischen Behandlung eines Auges, das refraktive Lasersystem aufweisend:

  - eine Steuereinheit (30); und
  - ein Ein- und Ausgabegerät (38);

**dadurch gekennzeichnet, dass**

- das refraktive Lasersystem (10) ferner eine als Recheneinheit ausgebildete Planungsvorrichtung (36) gemäß einem der Ansprüche 1 bis 8 umfasst;
- dass die Planungsvorrichtung (36) mit dem Ein- und Ausgabegerät (38) verbunden ist; und
- dass zudem die Steuereinheit (30) in die Recheneinheit integriert ist oder durch die Recheneinheit ausgebildet ist oder die Steuereinheit (30) separat von der Planungsvorrichtung (36) ausgebildet und mit der Planungsvorrichtung (36) verbunden ist.

10. Refraktives Lasersystem (10) gemäß Anspruch 9, ferner aufweisend:

- zumindest einen Femtosekundenlaser, vorzugsweise zum refraktiven chirurgischen Herauslösen eines Lentikels aus der Kornea im Rahmen eines SMILE Verfahrens.

11. Refraktives Lasersystem (10) gemäß einem der Ansprüche 9 und 10, ferner aufweisend:

- zumindest einen Excimer-Laser, vorzugsweise zur Ablation der Kornea und/oder zur Bearbeitung der Hornhaut unter einem temporär beseitigten Flap im Rahmen eines LASIK Verfahrens.

12. Verfahren zur automatisierten Planung einer refraktiven chirurgischen Behandlung eines Auges mittels eines refraktiven Lasersystems (10), das Verfahren umfassend die Schritte:

- Planen der refraktiven chirurgischen Behandlung des Auges auf Basis von vorbestimmten Eigenschaften des Auges;
- Berechnen zumindest einer volumetrischen Kenngröße der Kornea des Auges unter Verwendung der vorbestimmten Eigenschaften des Auges, wobei die volumetrische Kenngröße eine Auswirkung der geplanten refraktiven chirurgischen Behandlung auf das Auge charakterisiert;
- Berechnen einer zu erwartenden Volumenänderung der Kornea aufgrund der geplanten refraktiven chirurgischen Behandlung des Auges;

**dadurch gekennzeichnet, dass** das Verfahren ferner umfasst:

- Bestimmen einer Percent-Volume-Altered Kennzahl, PVA Kennzahl, aus der berechneten, zu erwartenden Volumenänderung der Kornea; und
- Ausgeben der zu erwartenden Volumenänderung der Kornea und/oder einer darauf basierenden Information, insbesondere der PVA Kennzahl, an einen Benutzer.

13. Verfahren gemäß Anspruch 12, wobei das Berechnen der zu erwartenden Volumenänderung der Kornea ein Berechnen einer anteilsmäßigen Volumenänderung und/oder einer prozentualen Volumenänderung des Volumens der Kornea umfasst.

14. Computerprogrammprodukt, umfassend Befehle, die bei der Ausführung des Programms durch eine Recheneinheit diese veranlassen, ein Verfahren gemäß Anspruch 12 oder 13 auszuführen.

15. Computerlesbares Speichermedium, umfassend Befehle, die bei der Ausführung des Programms durch eine Recheneinheit diese veranlassen, ein Verfahren gemäß Anspruch 12 oder 13 auszuführen.

**Claims**

1. Planning device (36) for automated planning of a refractive surgical treatment of an eye by means of a refractive laser system (10), wherein the planning device (36) is configured to perform the following steps:

- planning the refractive surgical treatment of the eye on the basis of predetermined properties of the eye;
- calculating at least one volumetric parameter of the cornea of the eye using the predetermined properties of the eye, the volumetric parameter characterizing an effect of the planned refractive surgical treatment on the eye;
- calculating an expected volume change of the cornea on account of the planned refractive surgical treatment of the eye;

**characterized in that** the planning device (36) is further configured to perform the following steps:

- determining a percent volume altered number, PVA number, from the calculated expected volume change of the cornea; and
- outputting the PVA number to a user of the refractive laser system and/or planning device.

2. Planning device according to Claim 1, wherein the calculation of the expected volume change of the cornea comprises a calculation of a proportional volume change and/or a percentage volume change of the volume of the cornea.

3. Planning device (36) according to Claim 1 or 2, wherein the at least one volumetric parameter is based on at least one of the following volume elements:

   - a flap volume;
   - a volume ablated and/or excised from the cornea;
   - a volume detached from the cornea or ablated and/or excised from the cornea and weighted according to its strength;
   - a partial volume of the epithelium and/or stroma and/or endothelium ablated and/or excised from the cornea or detached from the cornea;
   - a volume detached from the cornea;
   - a lenticular volume;
   - a volume implanted in the cornea.

4. Planning device according to any of the preceding claims, wherein the planning of the refractive surgical treatment of the eye on the basis of predetermined properties of the eye comprises the planning of a refractive surgical detachment of a lenticule from the cornea within the scope of a SMILE method.

5. Planning device (36) according to any of the preceding claims, wherein the predetermined properties of the eye comprise at least patient-specific state data of the cornea of the eye, and wherein the patient-specific state data optionally comprise a diameter of the cornea and/or a radius of curvature of the cornea and/or a strength of the cornea and/or an epithelial profile of the cornea.

6. Planning device (36) according to Claim 5, wherein the patient-specific state data comprise a thickness profile of the cornea.

7. Planning device (36) according to any of the preceding claims, wherein the planning device (36) is configured to further perform the following step:

   - in automated fashion, assessing the refractive surgical treatment of the eye on the basis of the at least one volumetric parameter; and
   - providing a result of automated assessment of the refractive surgical treatment of the eye to a user of the refractive laser system and/or planning device.

8. Planning device (36) according to Claim 7, wherein the automated assessment of the refractive surgical treatment preferably comprises an assessment of a risk to the eye on account of a change in the volume of the cornea due to the refractive surgical treatment of the eye, and wherein the planning device (36) is optionally configured to further perform one or more of the following steps:

   - outputting to the user of the refractive laser system (10) and/or planning device (36) information based on the assessed risk;
   - in automated fashion, adapting the planned refractive surgical treatment to reduce the risk and/or proposing an adaptation to the planned refractive surgical treatment to reduce the risk, should the risk exceed a first predetermined level;
   - preventing the refractive surgical treatment from being performed should the assessed risk exceed a second predetermined level.

9. Refractive laser system (10) for refractive surgical treatment of an eye, the refractive laser system comprising:

   - a control unit (30); and
   - input and output equipment (38);

**characterized in that**

- the refractive laser system (10) further comprises a planning device (36) according to any of Claims 1 to 8 that takes the form of a computing unit;
- **in that** the planning device (36) is connected to the input and output equipment (38); and
- **in that**, moreover, the control unit (30) is integrated into the computing unit or formed by the computing unit, or the control unit (30) is formed separately from the planning device (36) and connected to the planning device (36).

10. Refractive laser system (10) according to Claim 9, further comprising:

- at least one femtosecond laser, preferably for the refractive surgical detachment of a lenticule from the cornea within the scope of a SMILE method.

11. Refractive laser system (10) according to either of Claims 9 and 10, further comprising:

- at least one excimer laser, preferably for ablation of the cornea and/or for processing the cornea under a temporarily removed flap within the scope of a LASIK procedure.

12. Method for automated planning of a refractive surgical treatment of an eye by means of a refractive laser system (10), the method comprising the steps of:

- planning the refractive surgical treatment of the eye on the basis of predetermined properties of the eye;
- calculating at least one volumetric parameter of the cornea of the eye using the predetermined properties of the eye, the volumetric parameter characterizing an effect of the planned refractive surgical treatment on the eye;
- calculating an expected volume change of the cornea on account of the planned refractive surgical treatment of the eye;

**characterized in that** the method further comprises:

- determining a percent volume altered number, PVA number, from the calculated expected volume change of the cornea; and
- outputting the expected volume change of the cornea and/or information based thereon, in particular the PVA number, to a user.

13. Method according to Claim 12, wherein the calculation of the expected volume change of the cornea comprises a calculation of a proportional volume change and/or a percentage volume change of the volume of the cornea.

14. Computer program product comprising instructions that, when the program is executed by a computing unit, cause said computing unit to carry out a method according to Claim 12 or 13.

15. Computer-readable storage medium comprising instructions that, when the program is executed by a computing unit, cause said computing unit to carry out a method according to Claim 12 or 13.

**Revendications**

1. Dispositif de planification (36) pour la planification automatisée d'un traitement chirurgical réfractif d'un œil au moyen d'un système laser réfractif (10), le dispositif de planification (36) étant conçu de façon à mettre en œuvre les étapes suivantes :

- planifier le traitement chirurgical réfractif de l'œil en fonction de propriétés prédéterminées de l'œil ;
- calculer au moins une caractéristique volumétrique de la cornée de l'œil en utilisant les propriétés prédéterminées de l'œil, la caractéristique volumétrique caractérisant un effet du traitement chirurgical réfractif planifié sur l'œil ;
- calculer un changement de volume attendu de la cornée dû au traitement chirurgical réfractif planifié de l'œil ;

**caractérisé en ce que** le dispositif de planification (36) est en outre conçu pour mettre en œuvre les étapes suivantes :

- déterminer un indice de pourcentage de modification de volume, indice PVA, à partir du changement de volume calculé et attendu de la cornée ; et
- transmettre l'indice PVA à un utilisateur du système laser réfractif et/ou du dispositif de planification.

2. Dispositif de planification selon la revendication 1, dans lequel le calcul du changement du volume attendu de la cornée comprend le calcul d'un changement du volume proportionnel et/ou d'un changement du volume en pourcentage du volume de la cornée.

3. Dispositif de planification (36) selon la revendication 1 ou la revendication 2, dans lequel ladite au moins une caractéristique volumétrique est basée sur au moins un des éléments de volume suivants :

- un volume de volet, dit « flap » ;
- un volume ablaté et/ou excisé de la cornée ;
- un volume détaché de la cornée ou un volume ablaté et/ou excisé de la cornée, pondéré en fonction de sa solidité ;
- un volume partiel de l'épithélium et/ou du stroma et/ou de l'endothélium ablaté et/ou excisé de la cornée ou détaché de la cornée ;
- un volume détaché de la cornée ;
- un volume de lenticule ;
- un volume implanté dans la cornée.

4. Dispositif de planification selon l'une des revendications précédentes, dans lequel la planification du traitement chirurgical réfractif de l'œil en fonction de propriétés prédéterminées de l'œil comprend la planification d'une extraction chirurgicale réfractive d'un lenticule hors de la cornée dans le cadre d'une procédure SMILE.

5. Dispositif de planification (36) selon l'une des revendications précédentes, dans lequel les propriétés prédéterminées de l'œil comprennent au moins des données d'état de la cornée de l'œil spécifiques au patient et dans lequel les données d'état spécifiques au patient comprennent en option un diamètre de la cornée et/ou un rayon de courbure de la cornée et/ou une solidité de la cornée et/ou un profil épithélial de la cornée.

6. Dispositif de planification (36) selon la revendication 5, les données d'état spécifiques au patient comprenant un profil d'épaisseur de la cornée.

7. Dispositif de planification (36) selon l'une des revendications précédentes, le dispositif de planification (36) étant en outre conçu de façon à mettre en œuvre les étapes suivantes :

- évaluer automatiquement le traitement chirurgical réfractif de l'œil en fonction d'au moins une caractéristique volumétrique ; et
- fournir à un utilisateur du système laser réfractif et/ou du dispositif de planification un résultat de l'évaluation automatique du traitement chirurgical réfractif de l'œil.

8. Dispositif de planification (36) selon la revendication 7, dans lequel l'évaluation automatisée du traitement chirurgical réfractif comprend de préférence une évaluation d'un risque pour l'œil dû à un changement du volume de la cornée résultant du traitement chirurgical réfractif de l'œil, et dans lequel le dispositif de planification (36) est en outre optionnellement conçu pour mettre en œuvre une ou plusieurs des étapes suivantes :

- fournir à l'utilisateur du système laser réfractif (10) et/ou du dispositif de planification (36) des informations basées sur le risque évalué ;
- adapter automatiquement le traitement chirurgical réfractif prévu pour réduire le risque et/ou proposer une adaptation du traitement chirurgical réfractif prévu pour réduire le risque, si le risque dépasse un premier niveau prédéterminé ;
- empêcher la réalisation du traitement chirurgical réfractif si le risque évalué dépasse un deuxième niveau prédéterminé.

9. Système laser réfractif (10) pour le traitement chirurgical réfractif d'un œil, le système laser réfractif comprenant :

- une unité de commande (30) ; et
- un appareil d'entrée et de sortie (38) ;

**caractérisé en ce que**

- le système laser réfractif (10) comprend en outre un dispositif de planification (36) conçu sous la forme d'une unité de calcul selon l'une des revendications 1 à 8 ;
- le dispositif de planification (36) est relié à l'appareil d'entrée et de sortie (38) ; et
- l'unité de commande (30) est en outre intégrée dans l'unité de calcul ou est formée par l'unité de calcul ou l'unité de commande (30) est formée séparément du dispositif de planification (36) et est reliée au dispositif de planification (36).

**10.** Système laser réfractif (10) selon la revendication 9, comprenant en outre :

- au moins un laser femtoseconde, de préférence pour l'ablation chirurgicale réfractive d'un lenticule de la cornée dans le cadre d'une procédure SMILE.

**11.** Système laser réfractif (10) selon l'une des revendications 9 et 10, comprenant en outre :

- au moins un laser excimer, de préférence pour l'ablation de la cornée et/ou pour le traitement de la cornée sous un volet temporairement retiré dans le cadre d'une procédure LASIK.

**12.** Procédé de planification automatisée d'un traitement chirurgical réfractif d'un œil au moyen d'un système laser réfractif (10), le procédé comprenant les étapes suivantes :

- planifier le traitement chirurgical réfractif de l'œil en fonction de caractéristiques prédéterminées de l'œil ;
- calculer au moins une caractéristique volumétrique de la cornée de l'œil en utilisant les propriétés prédéterminées de l'œil, la caractéristique volumétrique caractérisant un effet du traitement chirurgical réfractif planifié sur l'œil ;
- calculer un changement de volume attendu de la cornée dû au traitement chirurgical réfractif prévu de l'œil ;

**caractérisé en ce que** le procédé comprend en outre :

- le fait de déterminer un indice de pourcentage de modification de volume, indice PVA, à partir du changement de volume calculé et attendu de la cornée ; et
- le fait de transmettre à un utilisateur le changement du volume attendu de la cornée et/ou d'une information basée sur celle-ci, en particulier l'indice PVA.

**13.** Procédé selon la revendication 12, dans lequel le calcul du changement du volume attendu de la cornée comprend le calcul d'un changement du volume proportionnel et/ou d'un changement du volume en pourcentage du volume de la cornée.

**14.** Programme informatique comprenant des instructions qui, lors de l'exécution du programme par une unité de calcul, l'amènent à mettre en œuvre un procédé selon la revendication 12 ou la revendication 13.

**15.** Support de stockage lisible par ordinateur comprenant des instructions qui, lors de l'exécution du programme par une unité de calcul, amènent l'unité de calcul à mettre en œuvre un procédé selon la revendication 12 ou la revendication 13.

Fig. 1

$V_L =$

$V_1$

$+$

$V_2$

$-$

$V_3$

## Fig. 2A

$R_{CA}$

$R_F$

$d_F$

$R_L$

$V_L$

$h_L$  $h_F$

$D_L$

## Fig. 2B

Fig. 3A

Fig. 3B

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008028509 A1
- EP 1719483 A1
- DE 102011083928 A1
- DE 102015218909 A1

**In der Beschreibung aufgeführte Nicht-Patentliteratur**

- **VON D. GANTIEL et al.** Comparison of the effect of LASIK parameters on the percent tissue altered (1-dimensionalmetric) versus percent volume altered (3-dimensional metric). *J Cataract Refract Surg*, 2018, vol. 44, 897-904